# EUROPEAN PATENT SPECIFICATION

(11) **EP 3 515 387 B1**
(45) Date of publication and mention of the grant of the patent: **22.07.2020**
(21) Application number: 17777194.6
(22) Date of filing: 15.09.2017
(51) Int. Cl.: A61F 13/511, A61F 13/84

(54) **ABSORBENT ARTICLE WITH PH CONTROLLED TOPSHEET**
SAUGFÄHIGER ARTIKEL MIT PH-GESTEUERTER DECKSCHICHT
ARTICLE ABSORBANT AVEC FEUILLE SUPÉRIEURE À PH CONTRÔLÉ

(30) Priority: 22.09.2016 SE 1651257
(43) Date of publication of application: 31.07.2019
(73) Proprietor: Essity Hygiene and Health Aktiebolag, 405 03 Göteborg (SE)
(72) Inventor: STRIDFELDT, Chatrine, 405 03 Göteborg (SE); HUSMARK, Ulrika, 405 03 Göteborg (SE)
(74) Representative: Essity Hygiene and Health AB
(86) International application number: PCT/EP2017/073251
(87) International publication number: WO 2018/054766

(56) References cited:
- EP-A1- 1 245 240
- EP-A1- 1 358 894
- WO-A1-2014/193280
- WO-A1-2015/094067
- JP-A- 2007 138 325
- US-A1- 2016 051 423

## Description

### TECHNICAL FIELD

The disclosure relates to a disposable hygiene absorbent article, such as a sanitary napkin, a panty liner, a diaper or an incontinence pad, comprising a topsheet, an absorbent core and a backsheet, the topsheet being formed from a nonwoven web material comprising a dry coating comprising a pH control composition.

### BACKGROUND

Absorbent articles of the kind to which this disclosure relates are worn against the skin and comprise a topsheet, an absorbent core and a backsheet layer. All uses of products which are applied in direct contact with the skin may lead to unwanted side-effects. These side-effects may occur as a result of occlusion, moisture, mechanical, microbial, and enzymatic factors which all, to different degrees, interact and amplify the influence of each other and may cause different forms of skin irritation and primary or secondary skin infections which sometimes occur in users of said articles. An increase in pH is a normal phenomenon during use of sanitary articles in contact with skin. It has been demonstrated that skin with pH values below 5.0 is in a better condition than skin with pH values above 5.0. This has been shown by measuring the biophysical parameters of barrier function, moisturization and scaling. The effect of pH on adhesion of resident skin microflora has also been assessed and it has been shown that an acid skin pH (4-4.5) keeps the resident bacterial flora attached to the skin, whereas an alkaline pH (8-9) promotes the dispersal from the skin. See Lambers, H et al (2006) Natural skin surface pH is on average below 5, which is beneficial for its resident flora. Int J Cosmet Sci, 28 (5), 359-70.

Another example of unwanted effects of using absorbent articles in contact with the skin is the increased activity of enzymes such as lipases and proteases which exhibit a strongly pH-dependent activity that increases with increasing pH. With the increased enzyme activity, the skin starts to decompose and becomes sensitive to mechanical forces and bacterial attacks.

Absorbent articles comprising acidifying agents incorporated to control the pH of the skin in contact with the sanitary article are generally known in the art. The acidifying agents may be applied in lotions and also in aqueous solutions. An absorbent article impregnated with a lotion may suffer from an impaired absorbency. The lotion application step may also lead to complications in a production process.

Application of the acidifying agent in an aqueous form may have less impact on the absorbency of the sanitary article. However, there are also other aspects to take into account in terms of efficacy of the added agent during the use of a sanitary article, a simple and well-functioning application process as well as ensuring a long shelf life, i.e. storage stability.

### SUMMARY

An absorbent article incorporating a pH controlling agent with an improved and prolonged skin beneficial effect may be obtained in accordance with claim 1.

According to a first aspect, the present disclosure relates to an absorbent article comprising a topsheet, an absorbent core and a backsheet. The topsheet comprises a dry coating comprising a) an organic acid, having a pKa value not exceeding 5.5, and/or a salt thereof, b) a surface-active agent; and c) a water-soluble polymer.

The combination of the surface-active agent, the organic acid, having a pKa value not exceeding 5.5, and/or a salt thereof with a water-soluble polymer has been found to provide a prolonged and improved skin protecting effect of the organic acid and/or the salt thereof.

According to a second aspect, the present disclosure relates to a liquid permeable nonwoven web material having a dry coating comprising a) an organic acid, having a pKa value not exceeding 5.5, and/or a salt thereof, b) a surface-active agent; and c) a water-soluble polymer.

According to a third aspect, the present disclosure relates to methods for producing the above referenced liquid permeable nonwoven web material.

According to a fourth aspect, the present disclosure relates to methods for producing the above referenced hygiene absorbent article.

### DETAILED DESCRIPTION

The present disclosure relates to a disposable hygiene absorbent article comprising a topsheet, an absorbent core and a backsheet. The topsheet comprises a dry coating comprising a) an organic acid, having a pKa value not exceeding 5.5, and/or a salt thereof, b) a surface-active agent and c) a water-soluble polymer.

The term "disposable hygiene absorbent article" refers to products that are placed against the skin of the wearer to absorb and contain body exudates, like urine, faeces and menstrual fluid, which articles can also be used to deliver pH controlling agents to these areas. The invention refers to disposable articles, which means articles that are not intended to be laundered or otherwise restored or reused as a sanitary article. Examples of disposable absorbent articles include feminine hygiene products such as sanitary napkins, panty liners, sanitary panties and feminine inserts; diapers and pant diapers for infants and incontinent adults; incontinence pads; diaper inserts and the like.

As used herein, the term "dry coating" refers to a coating being formed on a topsheet material by application of a composition being applied to the topsheet material in a liquid carrier being liquid in room temperature, such as an aqueous solution, followed by drying of the topsheet material and resulting in a dry coating formed on the topsheet material. A "dry" coating, as referred to herein, relates to a coating that has a water content not significantly exceeding the level of water being inevitable due to an equilibrium with the water content in the surrounding air.

The coating composition may be applied to the topsheet material by any suitable means including spraying, slot coating, kiss roll coating and/or soaking the material in a bath containing the coating composition. The coating may be performed in-line during assembly of the absorbent article. Alternatively, the topsheet material may be prepared separately and delivered as ready-to-use rolls to the absorbent article manufacturing plant. The coating may also be formed by a combination of the above methods.

Each one of the components of the coating composition, i.e. the surface-active agent and the organic acid and/or salt thereof and the water-soluble polymer may be applied by one of the methods including spraying, slot coating, kiss roll coating and/or soaking the material in a bath containing the coating composition above and the water-soluble polymer may be applied by a separate method chosen from for example spraying, slot coating, kiss roll coating and/or soaking the material in a bath containing the coating composition, and may be added onto the nonwoven web material individually in separate steps or together in one single step.

After the topsheet material has been wetted with the coating composition, the topsheet material is dried by e.g. guiding the topsheet material through a forced hot air oven or across a bank of infrared light or dielectric dryers or other conventional drying apparatuses as are known to the skilled person in the art. For some organic acids, such as for example lactic acid, it is important to ensure that the temperature during the drying step is kept at relatively low temperature to avoid evaporation of the organic acid. For lactic acid, for example, the temperature should be kept below the boiling temperature of around 120 °C.

The method for producing the liquid permeable nonwoven web material, having a dry coating comprises in general the steps of providing a liquid permeable nonwoven web material; applying the following to the nonwoven web: an organic acid, having a pKa value not exceeding 5.5 and/or a salt thereof; a surface-active agent; and a water-soluble polymer.

The components of the coating composition may be applied in any order to the nonwoven web material.

For example, all the components may be applied in one single liquid, preferably aqueous solution, i.e. comprising said organic acid and/or salt thereof, said surface-active agent and said water-soluble polymer.

Alternatively, a first liquid composition comprising said surface-active agent is applied to the nonwoven web material and subsequently, a second liquid composition, preferably aqueous composition, comprising said organic acid and/or salt thereof and said water-soluble polymer is applied to the nonwowen web material.

As a further alternative, the three components, i.e. the organic acid and/or salt thereof, the surface-active agent and the water-soluble polymer, are contained in separate liquid, preferably aqueous compositions, and are applied simultaneously, or subsequently to each other in any order, to the nonwoven web material. Preferably in such case, the surface-active agent is applied to the nonwoven web material prior to the application of the water-soluble polymer.

After application of the liquid coating compositions to the nonwoven web material, the so treated nonwoven web material is subjected to a drying step in so that a dry coating is formed on the nonwoven web material, which is then ready to be used as a topsheet material in accordance with the present disclosure.

A surface-active agent according to the present disclosure is a substance which lowers the surface tension of the medium in which it is dissolved, and/or the interfacial tension with other phases, and, accordingly, is positively adsorbed at the liquid/solid and/or at other interfaces. The surface-active agent may be any known surface-active agent suitable for use in hygienic applications, as is generally known in the art, suitable surface-active agents may include any cationic surfactant, anionic surfactant, nonionic surfactant, zwitterionic surfactant or surfactant of amine oxide type suitable for use in hygienic applications and should lower the surface tension of the aqueous solution to allow the solution to spread more easily over the topsheet. The surface-active agent may ensure an adequate spreading and impregnation of the coating in the topsheet material and promotes an improved adherence of the coating composition to the topsheet material. This is especially relevant for topsheet materials comprising a high amount of or entirely consisting of synthetic fibers and thus being mainly hydrophobic. A hydrophobic topsheet material may also be preferred due to the reduced wetting zone after wetting of the topsheet.

Examples on suitable surface-active agents are; Silastol PHP26, Silastol PHP 28, Silastol PHP 163, Silastol PHP 207 (Schill & Seilacher GmbH), Stantex S 6327 (Cognis), Duron OS 1547, Duron OF 4012 (CHT/BEZEMA), Nuwet 237* and Nuwet 550 (Momentive).

The organic acid, having a pKa value not exceeding 5.5, and/or a salt thereof is any or several of the following; lactic acid, a buffered solution of lactic acid, sodium lactate, potassium lactate and/or calcium lactate. Dried lactic acid and salts thereof have been found to form a smooth dry coating in contrast to other dried organic acids and salts thereof which may form crystals or a powdery composition when being dried in, for example, an aqueous solution. Such a smooth coating has been found to provide benefits in that the amount of active agents remaining on the topsheet after wetting is increased.

The function of the organic acid and/or salt thereof according to the present disclosure is to maintain natural skin pH and thus a beneficial intimate skin health. This may be achieved by the use of a sanitary article in accordance with the present disclosure since the organic acid and/or salt thereof is coated onto the topsheet which is the material layer being in direct contact with the skin of the user. It is believed that the transfer of the organic acid and/or salt thereof may occur both as dry transfer from the coating being in contact with the skin and as wet transfer by means of the bodily fluids being in contact with the coating and also with the skin and the environment between the topsheet and the user skin. Hence, by ensuring that as much as possible of the added organic acid and/or the salt thereof remains on and in the topsheet and is not dissolved and transferred down into the sanitary article, the effectiveness of the pH control composition is increased and prolonged.

A pH for the topsheet material of about 3,5 - 5 or a pH of about 4 - 4,5 has been proven advantageous to maintain a natural skin pH and thus a beneficial intimate skin health.

The topsheet may therefore comprise the organic acid having a pKa value not exceeding 5.5, and/or a salt thereof in an amount of at least about 0.1 g/m² of the topsheet, such as at least about 0.5 g/m² of the topsheet, such as at least about 1 g/m² of the topsheet. The organic acid and/or a salt thereof may be present in an amount from about 0.5 g/m² to about 10 g/m² of the topsheet, such as about 1 g/m² to about 10 g/m² of the topsheet.

The topsheet may also comprise a surface-active agent in an amount from at least about 0.1 % by weight of the topsheet, such as from about 0.1 to about 2 % by weight of the topsheet, such as from about 0.1 to about 1 % by weight of the topsheet, such as from about 0.3 to about 1 % by weight of the topsheet.

For the purposes of this disclosure, a weight percentage expressed in terms of "by weight of the topsheet" is to be calculated based on the total weight of the topsheet material in dry conditions, i.e. having reached equilibrium with surrounding air at 23°C at 50% RH.

An advantage of adding the organic acid and/or salt thereof in accordance with the present disclosure in conjunction with a surface-active agent is that it ensures good spreading and adherence to topsheet materials by lowering the surface tension of the liquid carrier, such as an aqueous solution, or of the topsheet material if applied as a first coating step.

The dry coating composition further comprises a water-soluble polymer in addition to the surface-active agent and the organic acid and/or salt thereof. Examples of such water-soluble polymers include polyacrylamide and copolymers thereof, polyvinyl alcohols, , polyalkylene glycols, polyamines, polyethyleneimines, polymeric quaternary ammonium compounds, polyvinylpyrrolidone and copolymers thereof, polyvinyl methyl ether/maleic anhydrides, polysaccharides and mixtures of two or more thereof. The dry coating composition does preferably not contain any polyacrylic acid and/or any copolymer thereof.

In a currently preferred embodiment, the water-soluble polymer is or comprises polyvinylpyrrolidone.

Even if the adherence of the dry coating to the topsheet material is improved by the presence of a surface-active agent, the dry coating composition may anyhow, to a certain extent, migrate away from the topsheet during storage. Without being bound by any theory, it is supposed that the organic acid tends to migrate towards the absorbent core. It has surprisingly been found that the combination of the organic acid and/or salt thereof and surface-active agent, with water-soluble polymer in accordance with the present disclosure, causes the low pH of the topsheet to be maintained for a prolonged time during storage, thereby prolonging the shelf life of the absorbent articles in accordance with the present disclosure.

Herein a polymer will be defined as water-soluble, if after adding 1 % by weight of polymer to ddH20 water with stirring at 25°C, adjustment of the pH to a value of 6-7, using NaOH or HCI as appropriate, and after further stirring for 1 hour at said temperature, at least 90% by weight of the product added is dissolved in the water.

The water-soluble polymers for use in the present disclosure may have a weight average molecular weight of from about 5,000 Da to 1,000,000 Da, such as from 10,000 to 500,000 Da, for example from 20,000 to 100,000 Da.

When the water-soluble polymer is, or comprises, a polyalkylene glycol, such as polyethylene glycol or polypropylene glycol, the weight average molecular weight of such polyalkylene glycol is from 5,000 to 1,000,000 Da.

The water-soluble polymer may be present in the topsheet dry coating in an amount of from 0.05 to 10, preferably from 0,1 to 5, more preferably from 0.3 to 3 g/m² of the topsheet.

The absorbent hygiene article according to this disclosure comprises an absorbent structure, the absorbent core, arranged between the topsheet and the backsheet. The absorbent core can be of any conventional kind. Examples of commonly occurring absorbent materials are cellulosic fluff pulp, tissue layers, highly absorbent polymers (so called superabsorbents), absorbent foam materials, absorbent nonwoven materials or the like. It is common to combine cellulosic fluff pulp with superabsorbents in an absorbent structure. It is also common to have absorbent structures comprising layers of different material with different properties with respect to liquid acquisition capacity, liquid distribution capacity and storage capacity. This is well-known to the person skilled in the art and does therefore not have to be described in detail. The thin absorbent bodies, which are common in today's sanitary articles, often comprise a compressed mixed or layered structure of cellulosic fluff pulp and superabsorbent. The size and absorbent capacity of the absorbent core may be varied to be suited for different uses such as sanitary napkins, pantyliners, adult incontinence pads and diapers, baby diapers, pant diapers, etc.

The liquid permeable topsheet can be any suitable topsheet material as known by the person skilled in the art and may be fibrous topsheet material composed of a nonwoven material, e g spunbonded, meltblown, carded, hydroentangled, wetlaid etc. Suitable nonwoven materials can be composed of natural fibers, such as woodpulp or cotton fibres, syntethic thermoplastic fibres, such as polyolefins, polyesters, polyamides and blends and combinations thereof or from a mixture of natural and syntethic fibres. Further examples of topsheet materials are porous foams. The materials suited as topsheet materials should be soft and non-irritating to the skin and be readily penetrated by body fluid, such as urine or menstrual fluid. The topsheet material may essentially constitute of non-absorbent fibers, such as synthetic thermoplastic fibers, such as such as polyolefins, polyesters, polyamides and blends and combinations thereof. The synthetic fibers may be monocomponent fibers, bicomponent fibers or multicomponent fibers comprising polyesters, polyamides and/or polyolefins such as polypropylene and polyethylene.

The topsheet may also have a basis weight of 22 g/m² and less, such as 20 g/m² and less, such as 19 g/m² and less. A topsheet having an open structure has been found to contribute to further reducing the wetting zone leading to a prolonged skin beneficial effect when used in a sanitary article in accordance with the present disclosure. The topsheet may furthermore be of a structure which do not promote wicking in X-Y direction, such as a uniform, even and non-corrugated structure.

The backsheet may consist of a thin plastic film, e.g. a polyethylene or polypropylene film, a nonwoven material coated with a liquid impervious material, a hydrophobic nonwoven material, which resists liquid penetration. Laminates of plastic films and nonwoven materials may also be used. The backsheet material is preferably vapour permeable, breathable, so as to allow vapor to escape from the absorbent structure, while still being liquid impermeable, preventing liquids from passing through the backsheet material.

The hygiene absorbent article in accordance with the present disclosure may comprise further layers in addition to the topsheet, backsheet and absorbent core, as is well known in the art. For example, an acquisition layer, preferably a porous fibrous layer essentially consisting of non-absorbent fibers may disposed between the absorbent core and the topsheet. The topsheet may also comprise or consist essentially of non-absorbent fibers.

The term "non-absorbent fibers" refers to fibers which do not absorb water to an appreciable extent. Suitable polymers from which the non-absorbent fibers may be formed are non-water-absorbent polymers such as polyolefins, polyesters, polyamides and blends and combinations thereof. The non-absorbent fibers may be monocomponent fibers, bicomponent fibers or multicomponent fibers comprising polyolefins, polyesters, polyamides and blends and combinations thereof.

By that the fibrous acquisition layer or the topsheet material "essentially" consist of non-absorbent fibers means that at least 95 % of the fibers are non-absorbent fibers, such as at least 99%, such as at least 100% of the fibers in the acquisition layer or the topsheet material are non-absorbent fibers. The acquisition layer and the topsheet material may however also include further substances present in small amounts, such as for example binders and pigments, as known by the person skilled in the art.

In order to improve retention of the organic acid having a pKa value not exceeding 5.5, and/or a salt thereof on/in the topsheet material and thus improve the skin beneficial effect thereof, it is advantageous to combine the topsheet comprising the dry coating composition with an acquisition layer which promotes a greater body contact surface having a low pH.

The presence of an acquisition layer in accordance with the present disclosure in contact with and underneath such a topsheet has been found to give a smaller wetting zone on the topsheet and thus a smaller zone in which the pH is raised compared to when the acquisition layer comprises absorbing fibers such as pulp fibers.

The thickness under load of the acquisition layer may be at least about 0.5 mm, such as at least about 1, such as at least about 1.5. The thickness under load of the acquisition layer may also be from about 0.5 to about 30 mm, and is measured according to WSP 120.6, according to method option A, wherein the thickness is measured after removing 1 layer of material from a slitted roll, the material being conditioned 30 minutes before measuring. The pressure used was 0.5 kPa with a presser-foot having an area of 25 cm² and with 10 s waiting before taking the thickness value.

The basis weight of the acquisition layer may be between at least 20 g/m², such as at least 40 g/m², such as from 40 g/m² to 150 g/m², such as from 40 g/m² to 100 g/m².

The thickness and the basis weight of the acquisition layer are of importance to ensure that the acquisition layer provides a sufficiently open and thick structure. This open, thick structure lacking intrinsic absorption capacity contributes to minimizing the wetting zone of the topsheet after wetting. It may also act as a non-absorbing reservoir for temporary liquid containment, which enables liquid in the acquisition layer to rewet to the topsheet to some extent whereby exchange of pH properties with the liquid on the topsheet surface may take place, which promotes a beneficial acidity on the topsheet surface.

### Test Method for measuring pH of the topsheet

The pH of the topsheet can be measured very precisely with the following method.

### Test Equipment

The pH meter used was PH-METER, VWR™ SYMPHONY, SB 80PI and the pH electrode used was HAMILTON FLATTRODE.

### Test Procedure

The pH meter rod was dipped in deionized water in the beginning of each new measurement, leaving a small droplet of water hanging from the electrode, which droplet was used to wet the nonwoven web material at the point of measurement. pH was measured at 23°C, at approx. 50% RH.

### Example 1 - pH stability

Nonwoven was produced with lactic acid and surface-active agent, with or without the water-soluble polymer polyvinyl pyrrolidone (PVP). The aim was to measure if storage stability could be improved with the addition of a water-soluble polymer.

Two cases were investigated. In the first case (sample 2) the buffered lactic acid and surface-active agent was added to the nonwoven in a first step and dried. Thereafter PVP was sprayed onto the nonwoven, and the resulting coated nonwoven was dried. In the second case (sample 3) the nonwoven was pretreated with surface-active agent and thereafter sprayed with a mix of buffered lactic acid and a water-soluble polymer (PVP and buffered lactic acid in one step).

Both these samples were compared to a reference (sample 1) which was a nonwoven with buffered lactic acid and wetting agent but with no polymer.

All these samples were put directly on top of an absorbent core comprising cellulose fibers and superabsorbent and stored in room temperature (covered with plastic trays). pH was measured directly after production and thereafter once a week for a duration of 6 weeks.

### Materials:

- Nonwoven 2:11: nonwoven with wetting agent (standard pp/ spun bond / 17 g/m², PHP26 cationic surfactant (approx. 0.15% by weight)), commercially available from Berry Plastic
- Nonwoven 2:3: nonwoven (standard pp/ spun bond /17 g/m²), commercially available from Berry Plastic, coated with buffered lactic acid (0.8 g dry weight/m²) and wetting agent PHP26 cationic surfactant (approx. 0.15% by weight)
- Buffered Lactic acid: (Lactic acid / Sodium Lactate, available from Corbion) was blended to a 1% w/w solution and pH of 3.2 measured.
- PVP: 40 kDa (M_{w}) polyvinylpyrrolidone dissolved in ddH20 to a 10% w/w solution.
- pH meter: VWR symphony SB80P1
- Absorbent core: 300g cellulose fibers and SAP 30%

| **Sample** | **Sample** | **Material description** |
|---|---|---|
| 1 (Comparative) | Ref 2:3 | Nonwoven 2:3. Buffered Lactic acid and wetting agent was blended and added in pilot scale to the nonwoven. The dry weight of Lactic acid buffer was calculated and measured to be approximately 0.8 g/m². |
| 2 | 2:3 + PVP | Nonwoven 2:3 was sprayed with the PVP solution to approximately 30 g/m² (which in dry weight corresponds to 3 g PVP/m².) |
| 3 | 2:11 + LA + PVP | Nonwoven 2:11 was sprayed with a blend of Lactic acid and PVP (90 ml buffered LA and 10 ml PVP). Also in this case was the total wet amount added approximately 30 g/m² (which in dry weight corresponds to 0.3 g PVP /m² and 0.3 g lactic acid buffer/m²). |

The nonwovens were dried in room temperature and thereafter put directly on the core of an absorbent product. pH was measured according to the method above directly and thereafter once a week. The products were stored covered with plastic trays in room temperature.

**Table 1: Test Results**

| | **Week** | **0** | **1** | **2** | **3** | **4** | **5** | **6** |
|---|---|---|---|---|---|---|---|---|
| **Sample** | | pH | | | | | | |
| **1** | | 3.7 | 5.4 | 5.4 | 5.3 | 5.4 | 5.7 | 5.8 |
| **2** | | 3.9 | 4.1 | 4.1 | 4.3 | 4.3 | 4.4 | 4.5 |
| **3** | | 3.3 | 3.8 | 4.1 | 4.1 | 4 | 4.3 | 4.1 |

As apparent from the table 1 above, The addition of a water-soluble polymer / PVP to nonwoven with Lactic acid significantly improved storage stability. This improvement was registered both when PVP was sprayed as a last step and when it was blended with buffered lactic acid and applied as a mix. We had good results for a total add on of both 0.3 g/m² and 3 g/m².

## Claims

1. A disposable hygiene absorbent article, comprising a liquid permeable topsheet, a backsheet and an absorbent core disposed between said topsheet and said backsheet, wherein said topsheet is of a nonwoven web material having a dry coating, said coating comprising
a. an organic acid, having a pKa value not exceeding 5.5 and/or a salt thereof, wherein said organic acid and/or salt thereof is selected from lactic acid, a buffered solution of lactic acid, sodium lactate, potassium lactate and calcium lactate or mixtures thereof;
b. a surface-active agent; and
c. a water-soluble polymer,
with the proviso that when said water-soluble polymer comprises a polyalkyleneglycol, said polyalkyleneglycol has a weight average molecular weight of from 5,000 to 1,000,000 Da

2. The article according to any one of the preceding claims, wherein said organic acid and/or a salt thereof is present in an amount of at least 0.1, preferably 0.1 to 10, more preferably 0.5 to 5 g/m² of the topsheet.

3. The article according to any one of the preceding claims, wherein said surface-active agent is present in an amount of at least 0.01%, preferably from 0.01 to 3%, more preferably from 0.1 to 1% by weight of the topsheet.

4. The article according to any one of the preceding claims, wherein said surface-active agent is selected from the group consisting of non-ionic surface-active agents, cationic surface-active agents, zwitterionic surface-active agents or amine oxide type surface-active agents, or mixtures thereof, preferably cationic surface-active agents.

5. The article according to any one of the preceding claims, wherein said water-soluble polymer does not contain any polyalkylene glycol, preferably no polyethylene glycol or polypropylene glycol, and/or does not contain any polyacrylic acid or copolymer of polyacrylic acid

6. The article according to any one of the preceding claims, wherein said water-soluble polymer is selected from the group consisting of polyacrylamide and copolymers thereof, polyvinyl alcohols, polyalkylene glycols, polyamines, polyethyleneimines, polymeric quaternary ammonium compounds, polyvinylpyrrolidone and copolymers thereof, polyvinyl methyl ether/maleic anhydrides, polysaccharides and combinations of two or more thereof.

7. The article according to any one of the preceding claims, wherein said water-soluble polymer is a homopolymer or copolymer of vinylpyrrolidone.

8. The article according to any one of the preceding claims, wherein said water-soluble polymer has a weight average molecular weight of from 5,000 to 1,000,000 Da, preferably from 10,000 to 500,000 Da, more preferably from 20,000 to 100,000 Da.

9. The article according to any one of the preceding claims, wherein said water-soluble polymer is present in an amount of from 0.05 to 10, preferably from 0,1 to 5, more preferably from 0.3 to 3 g/m² of the topsheet.

10. A liquid permeable nonwoven web material having a dry coating, said coating comprising
a. an organic acid, having a pKa value not exceeding 5.5 and/or a salt thereof, wherein said organic acid and/or salt thereof is selected from lactic acid, a buffered solution of lactic acid, sodium lactate, potassium lactate and calcium lactate or mixtures thereof;
b. a surface-active agent; and
c. a water-soluble polymer, with the proviso that when said water-soluble polymer is a polyalkyleneglycol, said polyalkyleneglycol has a weight average molecular weight of from 5,000 to 1,000,000 Da.

11. A method for producing a liquid permeable nonwoven web material having a dry coating, comprising the steps of:
a. providing a liquid permeable nonwoven web material
b. applying the following to the nonwoven web:
i. an organic acid, having a pKa value not exceeding 5.5 and/or a salt thereof, wherein said organic acid and/or salt thereof is selected from lactic acid, a buffered solution of lactic acid, sodium lactate, potassium lactate and calcium lactate or mixtures thereof;
ii. a surface-active agent; and
iii. a water-soluble polymer, with the proviso that when said water-soluble polymer is a polyalkyleneglycol, said polyalkyleneglycol has a weight average molecular weight of from 5,000 to 1,000,000 Da.

12. The method according to claim 11, wherein step b) comprises applying one or more liquid, preferably aqueous, composition(s) of the respective compound onto the nonwoven web material, followed by a step c) of drying the so treated nonwoven web material.

13. The method according to claim 11 or 12, wherein step b) comprises applying an aqueous composition comprising said organic acid and/or salt thereof, said surface-active agent and said water-soluble polymer to the nonwoven web material; or wherein step b) comprises applying a first liquid composition comprising said surface-active agent to said nonwoven web material and subsequently applying a second liquid composition comprising said organic acid and/or salt thereof and said water-soluble polymer to the nonwoven web material; or wherein step b) comprises applying a first liquid composition comprising said surface-active agent and said organic acid and/or salt thereof to said nonwoven web material and subsequently applying a second liquid composition comprising water-soluble polymer to said nonwoven material.

14. A method for producing a disposable hygiene absorbent article as defined in any one of the claims 1 to 9, comprising the steps of
a. providing a liquid permeable nonwoven web material having a dry coating as defined in claim 10 or obtained according to claims 11 to 13; and
b. disposing an absorbent core between said nonwoven web material and a back sheet material.

15. The use of a liquid permeable nonwoven web material as defined in claim 10 or obtainable from the method of claims 11 to 13, as a topsheet in a disposable hygiene absorbent article.

## Patentansprüche

1. Saugfähiger Wegwerfhygieneartikel, umfassend eine flüssigkeitsdurchlässige Deckschicht, eine Unterschicht und einen saugfähigen Kern, der zwischen der Deckschicht und der Unterschicht angeordnet ist, wobei die Deckschicht aus einem Vliesstoffmaterial ist, das eine trockene Beschichtung aufweist, wobei die Beschichtung Folgendes umfasst
a. eine organische Säure, die einen pKa Wert aufweist, der 5,5 nicht übersteigt, und/oder ein Salz davon, wobei die organische Säure und/oder das Salz davon aus Milchsäure, einer Pufferlösung von Milchsäure, Natriumlactat, Kaliumlactat und Calciumlactat oder Gemischen davon ausgewählt ist;
b. ein oberflächenaktives Mittel; und
c. ein wasserlösliches Polymer,
mit dem Vorbehalt, dass, wenn das wasserlösliche Polymer Polyalkylenglykol umfasst, das Polyalkylenglykol ein Gewichtsdurchschnittsmolekulargewicht von 5 000 bis 1 000 000 Da aufweist.

2. Artikel nach einem der vorstehenden Ansprüche, wobei die organische Säure und/oder ein Salz davon in einer Menge von mindestens 0,1, vorzugsweise von 0,1 bis 10, bevorzugter von 0,5 bis 5 g/m² der Deckschicht vorhanden ist.

3. Artikel nach einem der vorstehenden Ansprüche, wobei das oberflächenaktive Mittel in einer Menge von mindestens 0,01%, vorzugsweise von 0,01 bis 3%, bevorzugter von 0,1 bis 1 Gew.-% der Deckschicht vorhanden ist.

4. Artikel nach einem der vorstehenden Ansprüche, wobei das oberflächenaktive Mittel aus der Gruppe ausgewählt ist, die aus nicht ionischen oberflächenaktiven Mitteln, kationischen oberflächenaktiven Mitteln, zwitterionischen oberflächenaktiven Mitteln, oder aminoxidartigen oberflächenaktiven Mitteln, oder Gemischen davon, bevorzugt kationischen oberflächenaktiven Mitteln besteht.

5. Artikel nach einem der vorstehenden Ansprüche, wobei das wasserlösliche Polymer kein Polyalkylenglykol, vorzugsweise kein Polyethylenglykol oder Polypropylenglykol, enthält, und/oder keine Polyacrylsäure oder Copolymer einer Polyacrylsäure enthält.

6. Artikel nach einem der vorstehenden Ansprüche, wobei das wasserlösliche Polymer aus der Gruppe ausgewählt ist, die aus Polyacrylamid und Copolymeren davon, Polyvinylalkoholen, Polyalkylenglykolen, Polyaminen, Polyethyleneiminen, quaternären Polymerammoniumverbindungen, Polyvinylpyrrolidon und Copolymeren davon, Polyvinylmethylether/Maleinanhydriden, Polysacchariden und Kombinationen von zwei oder mehr davon besteht.

7. Artikel nach einem der vorstehenden Ansprüche, wobei das wasserlösliche Polymer ein Homopolymer oder Copolymer von Vinylpyrrolidon ist.

8. Artikel nach einem der vorstehenden Ansprüche, wobei das wasserlösliche Polymer ein Gewichtsdurchschnittsmolekulargewicht von 5 000 bis 1000 000 Da, vorzugsweise von 10 000 bis 500 000 Da, bevorzugter von 20 000 bis 100 000 Da aufweist.

9. Artikel nach einem der vorstehenden Ansprüche, wobei das wasserlösliche Polymer in einer Menge von 0,05 bis 10, vorzugsweise von 0,1 bis 5, bevorzugter von 0,3 bis 3 g/m² der Deckschicht vorhanden ist.

10. Flüssigkeitsdurchlässiges Vliesstoffmaterial, das eine trockene Beschichtung aufweist, wobei die Beschichtung Folgendes umfasst
a. eine organische Säure, die einen pKa Wert aufweist, der 5,5 nicht übersteigt, und/oder ein Salz davon, wobei die organische Säure und/oder das Salz davon aus Milchsäure, einer Pufferlösung von Milchsäure, Natriumlactat, Kaliumlactat und Calciumlactat oder Gemischen davon ausgewählt wird;
b. ein oberflächenaktives Mittel; und
c. ein wasserlösliches Polymer,
mit dem Vorbehalt, dass, wenn das wasserlösliche Polymer Polyalkylenglykol umfasst, das Polyalkylenglykol ein Gewichtsdurchschnittsmolekulargewicht von 5 000 bis 1 000 000 Da aufweist.

11. Verfahren zum Herstellen eines flüssigkeitsdurchlässigen Vliesstoffmaterials, das eine trockene Beschichtung aufweist, die folgenden Schritte umfassend:
a. Bereitstellen eines flüssigkeitsdurchlässigen Vliesstoffmaterials
b. Aufbringen des Folgenden auf den Vliesstoff:
i. eine organische Säure, die einen pKa Wert aufweist, der 5,5 nicht übersteigt, und/oder ein Salz davon, wobei die organische Säure und/oder das Salz davon aus Milchsäure, einer Pufferlösung von Milchsäure, Natriumlactat, Kaliumlactat und Calciumlactat oder Gemischen davon ausgewählt wird;
ii. ein oberflächenaktives Mittel; und
iii. ein wasserlösliches Polymer, mit dem Vorbehalt, dass, wenn das wasserlösliche Polymer Polyalkylenglykol umfasst, das Polyalkylenglykol ein Gewichtsdurchschnittsmolekulargewicht von 5 000 bis 1 000 000 Da aufweist.

12. Verfahren nach Anspruch 11, wobei Schritt b) Aufbringen einer oder mehrerer flüssiger, vorzugsweise wässriger Zusammensetzung(en) der entsprechenden Verbindung auf das Vliesstoffmaterial gefolgt von einem Schritt c) des Trocknens des so behandelten Vliesstoffmaterials umfasst.

13. Verfahren nach Anspruch 11 oder 12, wobei Schritt b) Aufbringen einer wässrigen Zusammensetzung, die die organische Säure und/oder ein Salz davon, das oberflächenaktive Mittel und das wasserlösliche Polymer umfasst, auf das Vliesstoffmaterial umfasst; oder wobei Schritt b) Aufbringen einer ersten flüssigen Zusammensetzung, das oberflächenaktive Mittel umfassend, auf das Vliesstoffmaterial, und anschließend Aufbringen einer zweiten flüssigen Zusammensetzung, die organische Säure und/oder ein Salz davon, und das wasserlösliche Polymer auf das Vliesstoffmaterial umfasst; oder wobei Schritt b) Aufbringen einer ersten flüssigen Zusammensetzung, das oberflächenaktive Mittel und die organische Säure und/oder ein Salz davon auf das Vliesstoffmaterial, und anschließend Aufbringen einer zweiten flüssigen Zusammensetzung, wasserlösliches Polymer umfassend, auf das Vliesmaterial umfasst.

14. Verfahren zum Herstellen eines saugfähigen Wegwerfhygieneartikels nach einem der Ansprüche 1 bis 9, die folgenden Schritte umfassend
a. Bereitstellen eines flüssigkeitsdurchlässigen Vliesstoffmaterials, das eine trockene Beschichtung nach Anspruch 10 aufweist oder nach den Ansprüchen 11 bis 13 erhalten wird; und
b. Anordnen eines saugfähigen Kerns zwischen dem Vliesstoffmaterial und einem Unterschichtmaterial.

15. Verwendung eines flüssigkeitsdurchlässigen Vliesstoffmaterials nach Anspruch 10 oder aus dem Verfahren nach den Ansprüchen 11 bis 13 erhältlich, als Deckschicht in einem saugfähigen Wegwerfhygieneartikel.

## Revendications

1. Article absorbant d'hygiène jetable, comprenant une feuille supérieure perméable aux liquides, une feuille arrière et une partie centrale absorbante disposée entre ladite feuille supérieure et ladite feuille arrière, ladite feuille supérieure étant constituée d'un matériau de tissu non tissé ayant un revêtement sec, ledit revêtement comprenant
a. un acide organique, ayant une valeur de pKa ne dépassant pas 5,5 et/ou un sel de celui-ci, dans lequel ledit acide organique et/ou un sel de celui-ci est choisi parmi l'acide lactique, une solution tamponnée d'acide lactique, le lactate de sodium, le lactate de potassium et le lactate de calcium ou des mélanges de ceux-ci ;
b. un agent tensioactif ; et
c. un polymère soluble dans l'eau,
à condition que lorsque ledit polymère soluble dans l'eau comprend un polyalkylèneglycol, ledit polyalkylèneglycol ait une masse moléculaire moyenne en poids de 5 000 à 1 000 000 Da

2. Article selon l'une quelconque des revendications précédentes, dans lequel ledit acide organique et/ou un sel de celui-ci est présent en une quantité d'au moins 0,1, de préférence de 0,1 à 10, plus préférentiellement de 0,5 à 5 g/m² de la feuille supérieure.

3. Article selon l'une quelconque des revendications précédentes, dans lequel ledit agent tensioactif est présent en une quantité d'au moins 0,01 %, de préférence de 0,01 à 3 %, plus préférentiellement de 0,1 à 1 % en poids de la feuille supérieure.

4. Article selon l'une quelconque des revendications précédentes, dans lequel ledit agent tensioactif est sélectionné à partir du groupe constitué par des agents tensioactifs non ioniques, des agents tensioactifs cationiques, des agents tensioactifs zwitterioniques ou des agents tensioactifs de type oxyde d'amine, ou leurs mélanges, de préférence des agents tensioactifs cationiques.

5. Article selon l'une quelconque des revendications précédentes, dans lequel ledit polymère soluble dans l'eau ne contient pas de polyalkylèneglycol, de préférence pas de polyéthylèneglycol ou de polypropylèneglycol, et/ou ne contient pas d'acide polyacrylique ou de copolymère d'acide polyacrylique.

6. Article selon l'une quelconque des revendications précédentes, dans lequel ledit polymère soluble dans l'eau est sélectionné à partir du groupe constitué par le polyacrylamide et ses copolymères, les alcools polyvinyliques, les polyalkylène glycols, les polyamines, les polyéthylèneimines, les composés d'ammonium quaternaire polymère, la polyvinylpyrrolidone et ses copolymères, le polyvinyle éther méthylique/anhydrides maléiques, les polysaccharides et combinaisons de deux ou plus de ceux-ci.

7. Article selon l'une quelconque des revendications précédentes, dans lequel ledit polymère soluble dans l'eau est un homopolymère ou un copolymère de vinylpyrrolidone.

8. Article selon l'une quelconque des revendications précédentes, dans lequel ledit polymère soluble dans l'eau a un poids moléculaire moyen de 5 000 à 1000 000 Da, de préférence de 10 000 à 500 000 Da, plus préférablement de 20 000 à 100 000 Da.

9. Article selon l'une quelconque des revendications précédentes, dans lequel ledit polymère soluble dans l'eau est présent en une quantité de 0,05 à 10, de préférence de 0,1 à 5, plus préférablement de 0,3 à 3 g/m² de la feuille supérieure.

10. Matériau de tissu non tissé perméable aux liquides ayant un revêtement sec, ledit revêtement comprenant
a. un acide organique, ayant une valeur de pKa ne dépassant pas 5,5 et/ou un sel de celui-ci, dans lequel ledit acide organique et/ou sel de celui-ci est choisi parmi l'acide lactique, une solution tamponnée d'acide lactique, le lactate de sodium, le lactate de potassium et le lactate de calcium ou des mélanges de ceux-ci ;
b. un agent tensioactif ; et
c. un polymère soluble dans l'eau, à condition que lorsque ledit polymère soluble dans l'eau est un polyalkylèneglycol, ledit polyalkylèneglycol ait une masse moléculaire moyenne en poids de 5 000 à 1 000 000 Da

11. Procédé de production d'un matériau de tissu non tissé perméable aux liquides ayant un revêtement sec, comprenant les étapes consistant à :
a. fournir un matériau de tissu non tissé perméable aux liquides
b. appliquer ce qui suit au tissu non tissé :
- i. un acide organique ayant une valeur de pKa ne dépassant pas 5,5 et/ou un sel de celui-ci, dans lequel ledit acide organique et/ou un sel de celui-ci est sélectionné parmi l'acide lactique, une solution tamponnée d'acide lactique, le lactate de sodium, le lactate de potassium et le lactate de calcium ou des mélanges de ceux-ci ;
- ii. un agent tensioactif ; et
- iii. un polymère soluble dans l'eau, à condition que lorsque ledit polymère soluble dans l'eau est un polyalkylèneglycol, ledit polyalkylèneglycol ait une masse moléculaire moyenne en poids de 5 000 à 1 000 000 Da.

12. Procédé selon la revendication 11, dans lequel l'étape b) comprend l'application d'une ou plusieurs compositions liquides, de préférence aqueuses, du composé respectif sur le matériau de tissu non tissé, suivie d'une étape c) de séchage du matériau de tissu non tissé ainsi traité.

13. Procédé selon la revendication 11 ou 12, dans lequel l'étape b) comprend l'application d'une composition aqueuse comprenant ledit acide organique et/ou un sel de celui-ci, ledit agent tensioactif et ledit polymère soluble dans l'eau au matériau de tissu non tissé ; ou dans lequel l'étape b) comprend l'application d'une première composition liquide comprenant ledit agent tensioactif sur ledit matériau de tissu non tissé et ensuite l'application d'une seconde composition liquide comprenant ledit acide organique et/ou un sel de celui-ci et ledit polymère soluble dans l'eau au matériau de tissu non tissé ; ou dans lequel l'étape b) comprend l'application d'une première composition liquide comprenant ledit agent tensioactif et ledit acide organique et/ou un sel de celui-ci sur ledit matériau de tissu non tissé et ensuite l'application d'une seconde composition liquide comprenant un polymère soluble dans l'eau sur ledit matériau non tissé.

14. Procédé de production d'un article absorbant d'hygiène jetable tel que défini dans l'une quelconque des revendications 1 à 9, comprenant les étapes consistant à
a. fournir un matériau de tissu non tissé perméable aux liquides ayant un revêtement sec tel que défini dans la revendication 10 ou obtenu selon les revendications 11 à 13 ; et
b. disposer une partie centrale absorbante entre ledit matériau de tissu non tissé et un matériau de feuille arrière.

15. Utilisation d'un matériau de tissu non tissé perméable aux liquides tel que défini dans la revendication 10 ou pouvant être obtenu à partir du procédé des revendications 11 à 13, en tant que feuille supérieure dans un article absorbant d'hygiène jetable.
